Europäisches Patentamt

European Patent Office

Office européen des brevets

(11) Publication number: **0 616 804 A1**

# EUROPEAN PATENT APPLICATION

(21) Application number: **94104577.5**

(22) Date of filing: **23.03.94**

(51) Int. Cl.5: **A61K 31/19**

(30) Priority: **26.03.93 IT MI930591**

(43) Date of publication of application:
**28.09.94 Bulletin 94/39**

(84) Designated Contracting States:
**AT BE CH DE DK ES FR GB GR IE IT LI LU MC NL PT SE**

(71) Applicant: **LABORATORIO FARMACEUTICO C.T. S.r.l.**
**Strada Solaro, 75-77**
**I-18038 Sanremo (Imperia) (IT)**

(72) Inventor: **Tessitore, Giuseppe**
**Via Dante Alighieri 73**
**I-18038 Sanremo (Imperia) (IT)**
Inventor: **Cacciaglia, Roberto**
**Via dei Pepi 60**
**I-18014 Ospedaletti (Imperia) (IT)**

(74) Representative: **Gervasi, Gemma, Dr. et al**
**NOTARBARTOLO & GERVASI Srl**
**Viale Bianca Maria 33**
**I-20122 Milan (IT)**

(54) **Gamma-hydroxybutyric acid salts possessing anxiolytic activity and suitable for the treatment of states of depression.**

(57) Use of pharmaceutically acceptable salts of gamma-hydroxybutyric acid as active ingredients for the preparation of pharmaceutical compositions possessing anxiolytic activity and suitable for the treatment of states of depression.

EP 0 616 804 A1

**Field of the invention**

The present invention relates to the use of pharmaceutically acceptable salts of gamma-hydroxybutyric acid as active ingredients for the preparation of pharmaceutical compositions possessing anxiolytic activity and suitable for the treatment of states of depression.

**State of the art**

As known, depression is an emotional disorder implying a state of lowered mood, characterized by deep sadness, pessimism, restlessness, self-pity, ideational poverty, and by organic symptoms, such as insomnia, anorexia, loss of enthusiasm and of libido.

Depression may be treated by the use of tricyclic antidepressants, monoamine oxidase inhibitors, some psychotropic drugs, lithium carbonate, and by electroconvulsive (ECS or electroshock) therapy ("Le basi farmaceutiche della terapia", Italian translation of the 6th U.S. edition of a text by Goodman and Gilman, pp. 440-456).

However, the treatment that proved to be most effective and, therefore, which is widely adopted, consists of the administration of tricyclic antidepressants, in particular imipramine.

This drug, like all other tricyclic antidepressants, is highly toxic, the median lethal dose ($LD_{50}$) values being 90 to 110 mg/kg i.p. and 400 to 490 mg/kg per os.

Most of the toxic reactions are referred to the antimuscarinic effects of said drugs and to their toxicity to the brain; however, also the effects on the heart constitute a serious problem (Goodmann and Gilman, ibid., pp. 439-456).

Another tricyclic antidepressant, mianserin, exhibits the following $LD_{50}$ values: mouse: 365 to 390 mg/kg per os and 31 to 32.5 mg/kg i.v.

As disclosed in U.S. patent No. 4.983.632, the pharmaceutically acceptable salts of gamma-hydroxybutyric acid and, in particular, the sodium salt thereof, are used to fight addiction to alcohol and to treat the relevant abstinence crisis.

**The present invention**

It has unexpectedly been found that the pharmaceutically acceptable salts of gamma-hydroxybutyric acid are particularly efficacious in the treatment of depression and possess anxiolytic activity, while their toxicity is much lower than that of the antidepressants and anxiolytics of commerce.

**Detailed description of the invention**

Preferred pharmaceutically acceptable salts of gamma-hydroxybutyric acid for use in the present compositions are lithium-, sodium-, potassium-, calcium-, and magnesium salts, the sodium salt being particularly preferred.

The following tests were carried out to check the activity of pharmaceutically acceptable gamma-hydroxybutyric acid salts in the states of depression:
- Despair test
- Learned helplessness
- Behavioural tests (as an anxiolytic drug).

**1. DESPAIR TEST**

This behavioural test developed by R.D. Porsolt et al. (Arch. int. Pharmacodyn. 229, 327-336, 1977) is commonly used to evaluate the activity of antidepressant agents.

The test is easy to carry out, requires very little apparatus, and shows excellent predictability characteristics: in fact, the antidepressants of the three main classe, i.e. tricyclic, monoamine oxidase inhibitors, and atypical, were positive by this test.

Furthermore, the potency of the tested drugs in clinical usage is strongly correlated with that observed in the behavioural test (Porsolt, 1977).

A further positive feature is the phenomenologic analogy between the animal immobility, which reflects its state of depression, and the symptoms sometimes noticed in depressive states in man, in particular in endogenous uni- and bipolar depression.

The animal does not show a generalized hypoactivity, but, rather, an inability to continue making efforts to escape. Similarly, depressed patients show serious psychomotor difficulties when subjected to tests requiring considerable efforts (Willner, Psychopharmacology (1984)83:1-16).

**Method**

Male Swiss mice (CD strain, Charles River) weighing 30 g were used and assigned at random to the various treatment groups.

The mice were plunged individually into a Plexiglas cylinder of 25 cm in height and 9 cm in diameter, filled with water at 24°C, for a 6 min latency up to 6 cm from the upper lip.

The duration of the test was 6 minutes. The time during which the animal kept floating passively was measured starting from the 2nd minute (duration of immobility).

One hour before testing, sodium gamma-hydroxybutyrate (NaGHB) as an acqueous suspension additioned of non-ionic surfactant Tween was administered by i.p. injection in a volume of 10 ml/kg, or per os.

Blind tests were performed. The statistical analysis of the data was performed, whenever possible, by Student's t-test, otherwise by Mann Whitney's non-parametric test.

The results of the despair test are shown in the following tables.

Table 1

| Results of despair tests on mice injected ip with NaGHB | | | |
|---|---|---|---|
| Dose (mg/kg) | Duration of immobility (s) | <% | P |
| 0 | 211 ± 16 | | |
| 5 | 179 ± 24 | 15 | 0.02 |
| 10 | 153 ± 19 | 27 | 0.02 |
| 20 | 134 ± 26 | 36 | 0.002 |
| 40 | 120 ± 12 | 43 | 0.0007 |

Table 2

| Results of despair tests on mice administered NaGHB per os | | | |
|---|---|---|---|
| Dose (mg/kg) | Duration of immobility (s) | <% | P |
| 0 | 229 ± 30 | | |
| 20 | 189 ± 23 | 17 | 0.05 |
| 50 | 137 ± 16 | 40 | 0.002 |
| 100 | 133 ± 31 | 42 | 0.002 |

**2. LEARNED HELPLESSNESS**

**Method**

The test method is as described by Seligman and Beagley in J. of Comparative and Physiological Psychology, Vol. 88, No. 2, 534-541, 1975.

Rats were subjected to a series of unescapable electric shocks to induce an artificial state of depression. Twenty-four hours later, the rats were put in a position to escape.

Despite said possibility, the shocked rats showed a deficit of escape as well as extreme passivity.

Vice versa, controls, which had not received electric shocks during phase 1, were reactive and their performances good.

The rats shocked during phase 1 and chronically treated (at least for 2 weeks) with known antidepressants showed a lower deficit of escape than the controls that had been shocked and treated with a single dose of physiological saline solution.

Once the test procedure had been developed, an experiment with NaGHB was carried out.

Male Sprague-Dawley rats initially weighing 200 g approx. were used.

The treatment, which consisted of a single daily i.p. injection, lasted 17 days. The two last injections were given 1 h before phase 1 and, respectively, 1 h before phase 2.

Positive control: 10 mg/kg imipramine hydrochloride.

The NaGHB dose administered was that found most reliable in rats submitted to the despair test after one-week treatment, i.e. 10 mg/kg.

The procedure consisted of two phases performed at a 24-h interval.

Phase 1: rats were confined to one section of the test apparatus, where they received 60 electric shocks (0.8 mA; 3 s) at approx. 20-sec intervals. Controls were confined to the other section for 30 min approx., but did not receive shocks.

Phase 2: this phase was preceded by a 5-min familiarization period, during which the door between the two sections was open.

The rats had thus the possibility of escaping into the other section.

The rats received 30 electric shocks (0.8 mA; 3 s) at 30-sec intervals.

The following parameters were evaluated:
- Number of unsuccessful escape attempts
- Mean escape reaction time

Rats were subdivided into four 8-rat groups, i.e.
- Controls (non-shocked in phase 1)
- LHC (shocked in phase 1)
- LHNaGHB (shocked in phase 1; 10 mg/kg NaGHB)
- CNaGHB (non-shocked in phase 1; 10 mg/kg NaGHB)

The results obtained are shown in Tables 3 and 4.

## Table 3

| Parameter | Controls | LHC | LHNaGHB | CNaGHB |
|---|---|---|---|---|
| No. of escape failures | 29 | 9 | 4 | 26 |
| | 30 | 1 | 25 | 21 |
| | 29 | 4 | 25 | 23 |
| | 29 | 26 | 30 | 19 |
| | 22 | 1 | 7 | 27 |
| | 30 | 25 | 30 | 19 |
| | 26 | 8 | 21 | 24 |
| | 28 | 11 | 28 | 24 |
| mean ± S.E. | 27.8±2.9 | 10.6±1.1 | 21.3±1.1 | 23.6±2.6 |
| P | | a) | b) | c) |
| | | 0.001 | 0.05 | 0.5 ns |

a) vs. controls

b) vs. LHC group (shocked controls)

c) vs. controls

4

## Table 4

| Parameter | Controls | LHC | LHNaGHB | CNaGHB |
|-----------|----------|-----|---------|--------|
| Mean reaction time | 5.47 | 6.49 | 5.29 | 4.91 |
| | 5.45 | 6.48 | 6.12 | 4.58 |
| | 4.56 | 6.51 | 6.21 | 4.64 |
| | 6.30 | 6.46 | 5.77 | 6.24 |
| | 4.73 | 6.11 | 5.88 | 4.46 |
| | 5.10 | 6.53 | 5.42 | 6.15 |
| | 5.72 | 6.26 | 5.60 | 4.83 |
| | 5.97 | | | |
| | No.8 | No.7 | No.7 | No.7 |
| mean ± S.E. | 5.41±0.6 | 6.4±0.16 | 5.76±0.34 | 5.4±1.0 |
| P | | a) | b) | c) |
| | | 0.001 | 0.02 | 0.98 ns |

a) vs. controls

b) vs. LHC group (shocked controls)

c) vs. controls.

Therefore, by comparing NaGHB-treated animals with shocked controls, the former show an increased number of attempts to escape and a decreased mean reaction time.

Finally, no significant difference was observed between the group of controls and the CNaGHB group, which proves the non-interference of the substance on non-shocked animals.

Also the datum of the CNaGHB group is interesting because the performances of the animals treated with gamma-hydroxybutyrate sodium salt are particularly good. This result might be explained by the drug anxiolytic activity, if any, which might accompany its antidepressive action. For example an anxiolytic action was documented for amitriptyline.

For this purpose, efforts were also made to develop the light/dark exploration test on the mouse.

## 3. LIGHT/DARK EXPLORATION TEST

The test is performed with a cage subdivided into two sections, a dark and a lit one.

Evidence was provided that the mice treated with adequate doses of anxiolytic drugs, e.g. Diazepam or Buspirone, spend a longer time in the lit section than controls.

The anxiolytic activity, if any, of the molecules can be evaluated on the basis of the time spent in the lit section.

The total duration of the test for each group of 8 animals was 4 hours. The results obtained are shown in Table 5 below.

5

Table 5

|  | Lit section | Dark section |
|---|---|---|
| Diazepam (10 mg/kg ip) | 25 | 7 |
| Buspirone (10 mg/kg ip) | 22 | 10 |
| NaGHB (10 mg/kg ip) | 23 | 9 |
| Controls | 18 | 14 |

The data are expressed as total hours (32) referred to all animals.

As may be seen, the anxiolytic activity of NaGHB is similar to that of Buspirone and Diazepam.

The toxicity of gamma-hydroxybutyrate sodium salt to rats compared with antidepressants and anxiolytics known to the prior art is shown in Table 6.

Table 6

| Substance | $LD_{50}$ (mg/kg) | Administration |
|---|---|---|
| NaGHB | 3,280 | ip |
| " | 12,360 | os |
| Imipramine | 90 - 110 | ip |
| " | 400 - 490 | os |
| Mianserin | 31 - 32.5 | ip |
| " | 365 - 390 | os |
| Diazepam | 300 | ip |
| " | 352 | os |
| Buspirone | 136 | ip |

The above results show that NaGHB is an effective antidepressant and anxiolytic and that its toxicity is much lower than that of the antidepressants and ansiolytics commonly used.

The pharmaceutically acceptable salt of gamma-hydroxybutyric acid is generally administered by the oral way, preferably in the form of single or multi-dose liquid solutions, or by injection, preferably by i.m. and by i.v. The pharmaceutical compositions for injection may also be buffered.

The compositions (per os and for injection) of the present invention preferably contain 20 to 500 mg active ingredient/dosage unit.

Some examples of pharmaceutical formulations containing NaGHB to be used as described in the present invention are conveyed by way of illustration and not of limitation.

**Formulation 1**

Single-dose 10 ml bottle

| **Active ingredient** | |
|---|---|
| 4-Hydroxybutyric acid sodium salt | 1.750 g |

| Excipients | |
| --- | --- |
| Glyceryl ammonium | 71.450 mg |
| Sodium saccharin | 35.700 mg |
| p-Hydroxymethylbenzoate | 7.500 mg |
| p-Hydroxypropylbenzoate | 2.500 mg |
| Sorbitol 70% | 7.000 ml |
| Sour cherry flavour | 0.035 ml |
| Purified water | q.s. |

**Formulation 2**

14-dose 140 ml bottle

| Active ingredient | |
| --- | --- |
| 4-Hydroxybutyric acid sodium salt | 24.500 g |

| Excipients | |
| --- | --- |
| Glyceryl ammonium | 1.000 g |
| Sodium saccharin | 0.500 g |
| p-Hydroxymethylbenzoate | 0.105 g |
| p-Hydroxypropylbenzoate | 0.035 mg |
| Sorbitol 70% | 98.000 ml |
| Sour cherry flavour | 0.490 ml |
| Purified water | q.s. |

**Formulation 3 for i.v. injection**

| 4-Hydroxybutyric acid sodium salt | 100 mg |
| --- | --- |
| Water, injection pure | 10.00 ml |

**Formulation 4 for i.m. injection**

| 4-Hydroxybutyric acid sodium salt | 150 mg |
| --- | --- |
| Citric acid | 3.00 mg |
| Sodium citrate | 10.00 mg |
| Water, injection pure | 4.00 ml |

**Claims**

1. Use of a pharmaceutically acceptable salt of gamma-hydroxybutyric acid as an active ingredient for the preparation of pharmaceutical compositions possessing anxiolytic activity and suitable for the treatment of states of depression.

2. The use according to claim 1, wherein said pharmaceutically acceptable salt of gamma-hydroxybutyric acid is selected among lithium-, sodium-, potassium-, calciun-, and magnesium salts.

3. The use according to claim 1, wherein said pharmaceutical compositions are suitable for oral administration, preferably in the form of single or multi-dose liquid solutions.

4. The use according to claim 1, wherein said pharmaceutical compositions are in the form of injectable formulations, which may also be buffered.

5. The use according to claim 1, wherein said pharmaceutical compositions contain 20 to 500 mg active ingredient/dosage unit.

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| X | RESEARCH COMMUNICATIONS IN PSYCHOLOGY, PSYCHIATRY AND BEHAVIOR<br>vol. 17, no. 3-4 , 1992<br>pages 109 - 122<br>R. ZERBIB ET AL. 'POTENTIAL ANTIDEPRESSANT ACTIVITY OF GAMMA-HYDROXYBUTYRATE IN THE MOUSE "BEHAVIORAL DESPAIR" TEST: CORRELATION WITH THE CENTRAL DOPAMINERGIC SYSTEM'<br>* the whole document *<br>--- | 1-5 | A61K31/19 |
| X | PRESSE MEDICALE<br>vol. 70, no. 47 , 1962<br>pages 2205 - 2207<br>DANON-BOILEAU ET AL. 'UTILISATION EN PSYCHIATRIE DU GAMMA-OH'<br>* the whole document *<br>--- | 1-5 | |
| X | BULLETIN OF EXPERIMENTAL BIOLOGY AND MEDICINE<br>vol. 77, no. 1 , 1974<br>pages 288 - 291<br>M. KRSIAK ET AL. 'EFFECT OF SODIUM HYDROXYBUTYRATE ON BEHAVIOR OF MICE AFTER PROLONGED ISOLATION'<br>* the whole document *<br>--- | 1-5 | **TECHNICAL FIELDS SEARCHED (Int.Cl.5)**<br><br>A61K |
| X | DIALOG FILE SUPPLIER: PASCAL AN=74-0015400 & BJULL. EKSPER. BIOL. MED.<br>vol. 77, no. 3 , 1974<br>pages 69 - 72<br>KRSHYAK ET AL. 'EFFET DE L'HYDROXYBUTYRATE DE SODIUM SUR LE COMPORTEMENT DES SOURIS AU COURS DE L'ISOLEMENT PROLONGE'<br>* the whole document *<br>--- | 1-5 | |

-/--

The present search report has been drawn up for all claims

| Place of search | Date of completion of the search | Examiner |
|---|---|---|
| THE HAGUE | 4 July 1994 | Hoff, P |

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| X | ACTIVITAS NERVOSA SUPERIOR vol. 15, no. 2 , 1973 pages 143 - 144 M. KRSIAK ET AL. 'EFFECT OF SODIUM GAMMA-HYDROXYBUTYRATE ON ISOLATION-INDUCED TIMIDITY IN MICE AND SLEEP IN RATS' * the whole document * --- | 1-5 | |
| X | DATABASE WPI Week 7802, Derwent Publications Ltd., London, GB; AN 78-03570A 'LITHIUM GAMMA HYDROXY-BUTYRATE USED FOR TREATMENT OF PSYCHOSIS AND SCHIZOPHRENIA' & SU-A-552 084 (AM USSR PHARMACOLOG) 15 April 1977 * abstract * --- | 1-5 | |
| X | GB-A-2 023 421 (GRISSMAN CHEMICALS LIMITED) 3 January 1980 * the whole document * --- | 1-5 | **TECHNICAL FIELDS SEARCHED** (Int.Cl.5) |
| X | ANNALI DI NEUROPSICHIATRIA E PSICOANALISI vol. 19, no. 1-4 , 1972 pages 150 - 157 F. MASTROSIMONE ET AL. 'CONSIDERAZIONI SULLE VARIAZIONI DEL VISSUTO TEMPORALE E SPAZIALE IN PATIENTI PSICHIATRICI IN CORSO DI TRATTAMENTO ANTIDEPRESSIVO CON GAMMA-OH' *abstract page 156* --- | 1-5 | |

-/--

The present search report has been drawn up for all claims

| Place of search | Date of completion of the search | Examiner |
|---|---|---|
| THE HAGUE | 4 July 1994 | Hoff, P |

EPO FORM 1503 03.82 (P04C01)

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| X | CHEMICAL ABSTRACTS, vol. 84, no. 19, 10 May 1976, Columbus, Ohio, US; abstract no. 130438u, 'TRANQUILLIZING PROPERTIES OF SODIUM HYDROXYBUTYRATE' * abstract * & BYULL. EKSP. BIOL. MED. vol. 81, no. 2 , 1976 pages 186 - 188 BUROV ET AL. | 1-5 | |

TECHNICAL FIELDS
SEARCHED     (Int.Cl.5)

The present search report has been drawn up for all claims

| Place of search | Date of completion of the search | Examiner |
|---|---|---|
| THE HAGUE | 4 July 1994 | Hoff, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)